# EUROPEAN PATENT SPECIFICATION

(11) **EP 2 173 220 B1**
(45) Date of publication and mention of the grant of the patent: **14.03.2012**
(21) Application number: 08774800.0
(22) Date of filing: 04.07.2008
(51) Int. Cl.: A47C 16/00, A61F 5/37

(54) **A CERVICAL SPINE AND NECK SUPPORT DEVICE**
VORRICHTUNG ZUM STÜTZEN VON HALSWIRBELSÄULE UND NACKEN
DISPOSITIF DE SUPPORT DE COLONNE CERVICALE ET DE COU

(30) Priority: 13.07.2007 IE 20070512; 15.05.2008 GB 0808868
(43) Date of publication of application: 14.04.2010
(73) Proprietor: Murnaghan, Shane, Greystones Wicklow (IE)
(72) Inventor: Murnaghan, Shane, Greystones Wicklow (IE)
(74) Representative: Catesby, Olivia Joanne
(86) International application number: PCT/EP2008/058724
(87) International publication number: WO 2009/010411

(56) References cited:
- GB-A- 2 179 852
- GB-A- 2 389 509
- US-A- 4 560 201
- US-A- 5 310 245
- US-A1- 2005 268 377

## Description

### Field of the Invention

The present invention relates to spinal supports. In particular the present invention relates to a spine and neck support device suitable for use as a sleeping aid for travellers.

### Background to the Invention

Travellers often find themselves in confined spaces with little room to manoeuvre. Often the seating provided on public transport, such as that provided on aeroplanes and buses lack a reclining function. In addition, foot space is increasingly limited. This limited foot space in addition to the confined upright position make journeys increasingly uncomfortable for passengers. Under such circumstances, it is difficult for passengers to relax and sleep. In particular, passengers are often forced to sleep without the aid of supine or prone reclining. The spine is designed to be held upright. Bending forward can stretch the lower back muscles to the point where they lose all their strength to protect from injury.

Sleeping without the aid of supine or prone reclining may lead to slouching or slumping, which results in a wide range of adaptations down the spine if the spine is not in a stable position. Similarly office workers, students, and those with desk based jobs often find themselves slumping at their desk forcing their spine into an unnatural and unstable position.

In certain circumstances when the spine is in an unstable position, the first rib locks in spasm causing rib problems such as rib dysfunction beneath the first thoracic vertebrae. The twelfth rib goes into distortion which due to it attachment and association to the diaphragm spasms repeatedly each time a person inhales and exhales. The Sacro Illac joints then shifts to facilitate the new posture. These changes cause muscular spasm at various sites from neck to pelvis. As the effects happen unconsciously, the person may wake to pain, mobility problems, and a need for medical attention. In turn this pain may lead to an undue reliance on painkillers and anti-inflammatory medications in an attempt to relieve the discomfort that has been caused by the inappropriate sleeping position. Additionally spines with stiff joints, damaged discs, or tight muscles may not get the nutrition needed due to the lack of movement, increasing the damage and reducing the speed of recovery from injury. There is a need in the art for a support that will prevent slumping while asleep by supporting the spine.

When a person's neck is pitched from a proper position, pressure accumulates on the cervical muscles and bones. The scale of pressure on the cervical muscles and bones accumulates massively the more a person's neck is pitched from it proper position. This compromised neck position can result in numerous problems.

Cranial based problems for example are often related to neck problems. Due to the combination of physical and psychic stress placed on the travellers confined to restricted areas trigger points, may occur in the myofascial tissue around the neck. Compromised sleeping positions, which alter the normal tone in muscles, fascia and other soft tissue can cause these trigger point to develop.

Trigger points are described as hyperirritable spots in skeletal muscle that are associated with palpable nodules in taut bands of muscle fibers. The palpable nodules are said to be small contraction knots and a common cause of pain. Compression of a trigger point may elicit local tenderness, referred pain, or motor dysfunction. Trigger points can in turn effect the extent of play in related joints, effect breathing and may also affect posture. The trigger points may restrict blood flow which may result in a local deficiency of blood supply. Therapeutic attention is usually required to remove the trigger point by massage. There is a need in the art for a means for maintaining the neck in it proper position and preventing the spine from unconscious slouching into positions that may generate trigger points or contribute to cranial based problems.

The muscles in the neck are complex and allow flexing, extending, rotating as well as side bending. In many cases travellers are restricted to confined areas with restricted leg space and insufficient space for lateral movement. Over long journeys, passengers are often forced to sleep in these confined areas. Under such conditions it is easy for an unassuming passenger to unconsciously orientate into bad postural positions. Bad posture is the most common cause of neck problems, for example muscular spasms. When the muscles of the neck go into spasm, a rotational force is applied to the ribs to which they are attached. This rotating action will in turn cause the vertebrae to enter into a "fixation" state wherein each vertebrae will spasm allowing virtually no movement of the columnar structure. In less severe cases, vertebral subluxation will occur where one or more of the vertebrae move out of position and create pressure on the spinal nerves. This results in restricted movement with possible pain. Very often remote sites in the neck hold the spasm pattern in the neck creating prolonged discomfort. Both "fixation" and "subluxation" require therapeutic attention.

Additional problems resulting from bad posture include the impingement of the Brachial Plexus. The Brachial Plexus is the system of nerves which serve the arm and emerge from the neck region. They emerge from the gap between the vertebrae and branch to the right and the left via the armpits. It contains 5 main nerves which cause numbness or tingling in the arm if compromised by bad posture. Some examples of the injuries that can occur due to the impingement of the Brachial Plexus include migraine, vertigo, shoulder stiffness, arm pain, lack of mobility, stroke symptoms, Bell's Palsy, ear pain, difficulty swallowing, sinus problems, blurred vision, neck pain, facial pain, breathing difficulties and back pain.

In the past many attempts have been made to facilitate the comfort of the travelling passenger. Support aids such as small pillows and inflatable supports that cup the neck have been used in an attempt to maintain the posture of the user. However these existing methods pose two problems in that a) the support they provide does not prevent the head falling forward on to the front of the chest and b) more importantly, none of the existing methods provide an anchor to stabilise the wearer's spinal symmetry. Other attempts to counteract back pain include back support chairs, however this method does not restrain the user in the correct postural position thereby allowing the users to crane their neck towards computer screens or slumping which renders the apparatus useless. However although these back support chairs may be suitable for the office environments they are unsuitable for installation on main stream public transport. Additionally they are an expensive alternative to standard office chairs and school chairs which although in many cases are ergonomically designed they do not stabilise the users spinal symmetry.

GB 2 389 509 discloses a head restraint adapted to be worn about the head, to support the head of the wearer that is sleeping or sitting upon a scat. The restraint comprises a headband or cap worn on the head of the wearer and a second part such as an apron attached onto the first part, where the second part is of a sufficient length to become trapped between the wearer's back and shoulders and the back of the seat the wearer is sat on. The preamble of appended claim 1 is based on this document.

US 4 560 201 provides a traveler's head support which includes an elongated relatively wide flexible woven strap having a relatively large loop formed at one end. The end of the loop is provided with a padded section for supporting the forehead of the user and an adjustable length strap attached across the top of the loop to hold the strap in position on the head of the user. The free end of the strap is of a proper length to extend down the back of the user and under an armpit and is wrapped or attached to one of the forearms_ GB 2 179 852 discloses a head support for seated persons comprising an adjustable strap for fitting securely about the forehead of the person and fixing means attached to the strap for fixing it permanently or temporarily to the back support or headrest of the seat at head height

US 2005/268377 provides a head restraint system for supporting the head of a person wearing headwear while seated. The head restraint is comprised of a headwear portion and a rear mounting portion. The headwear portion is comprised of an article of headwear with one part of a fastener attached at the rear of the headwear. The rear mounting portion comprises the other part of said fastener attached to the rear mount such that a user wearing the headwear may lean his or her head against the seat thereby engaging the parts of the fastener and intern restraining the users head.

US 5 310 245 discloses a cushion support for infants for use in cooperation with infant transport apparatus such as car seats, the support apparatus aiding in maintaining the infant's body in a proper posture. The apparatus is a T-shaped cushion member having a plurality of hook and loop fasteners, the T-shaped cushion member conformable to the shape of the car seat or baby seat, the hook and loop fasteners permitting the planar cushion to be rolled and secured to provide for lateral cushion support adjacent the infant's head and to permit the positioning of lateral cushion supports on the baby's sides.

There is a need in the art for an apparatus that will encourage proper posture in car seats to enhance the protection afforded by seatbelts. Particularly in the case of below average height adults or children using the adult safety restraints, slouching while asleep or awake can lead to increased levels of "scatbelt syndrome" as a result of accidents and collisions. For drivers of passenger vehicles or commercial vehicles, encouraging proper posture results in fewer injuries due to slouching or slumping.

There is a need in the art for an apparatus that will stabilise the users spinal symmetry holding the spine in its natural position (whether the user is awake or asleep) while additionally allowing the user to sleep without having to hold oneself up.

There is also a need in the art for a device that can be used as a training aid to encourage the user to maintain the correct postural position and to train the body's postural muscles.

### Object of the Invention

The object of the present invention is to provide a support to the spine at the cranial, cervical, thoracic and lumbar spine levels, including the spinal sacral ligament structure and coccyx structure.

It is an object of the present invention to provide a means that will facilitate the comfort of the travelling passenger.

It is further object of the present invention to provide a support that will prevent slumping while asleep by maintaining the neck in it proper position and preventing the spine from unconscious slouching into positions that may generate trigger points or contribute to cranial based problems.

It is further object of the present invention to provide a support that will stabilise the user's spinal symmetry holding the spine in its natural position while allowing the user to sleep in a sitting position without having to hold oneself up.

It is a further object of the present invention to increase the effectiveness of a car seatbelt by maintaining the seatbelt user in a position which greatly increases the harnessing effect of the seatbelt, and ensures that the harnessing effect of the seat belt is not compromised by the user slouching or sleeping.

It is a further object of the present invention to provide a training device to train a user's postural muscles and also to maintain the users posture in a correct position while working at a computer or desk.

### Summary of the Invention

The present invention provides a back flap for use in a cervical spine and neck support device, a cervical spine and neck support device incorporating same and a kit comprising multiple of said back flaps as described in appended independent claims 1, 2 and 10, respectively. Preferred embodiments are defined in the dependent claims. In particular, the cervical spine and neck support device for stabilising the spinal symmetry of a user when seated, may comprise a head garment comprising a band of material for surrounding a user's head, and a back flap comprising a head garment-cooperating end attachable to the head garment and a free end on which the user sits; wherein in use with the band of material surrounding the user's head and the head garment-cooperating end of the back flap attached to the head garment, the back flap is of sufficient length to allow the user to sit on the free end, in order to put the back flap under tension thereby restricting movement of the user's head and neck to stabilise the user's spine.

In one embodiment, the back flap is removable from the head garment. It will be appreciated that removing the back flap may allow for interchangeable flaps to be used with the same head garment. It will also be appreciated that the removable flap would also allow for ease of storage of the flap. In a further arrangement the head garment may be worn as a normal hat and the back flap attached in the required circumstances. Possible solutions for attaching the back flap to the head garment include buttons, clasps, clips, Velcro^{™}, or zips.

In an alternative embodiment the back flap is permanently attached to the head garment.

Preferably the head garment is a cap, such as a ball or a baseball cap, however it is not limited as such. It will be appreciated that visors, wrap-around sunglasses, peaked caps, or hats would also be suitable.

The cervical spine and neck support device may comprise means for storing the back flap when not in use. It will be appreciated that this arrangement may allow the user to normally wear the head garment and deploy the back flap when necessary.

The cervical spine and neck support device preferably comprises at least one tag on said back flap for aiding correct positioning of the back flap by the user prior to sitting down. Preferably at least one tag is provided on each edge of the flap. At least one tag may also be provided on the free end of the back flap. In one embodiment, the user can maintain tension in the back flap by holding the tags, allowing correct positioning of the back flap.

The head garment may be adjustable to fit the user's head. An adjustable strap such as a belt and buckle arrangement, elastic band or clasp may be provided. Alternatively at least the band of the head garment may be elasticised or otherwise stretchable. The entire head garment may be elasticised or stretchable.

The headband may also incorporate one or more comfort strip. In one embodiment the comfort strip is a forehead comfort strip, however it will be appreciated that the comfort strip may extend around the any part or all of the inner headband. It will also be appreciated that the comfort strip may also be used to ensure a snug fit for the head garment on the users head. This comfort strip may also be removable. The comfort strip may be a foam strip or a cotton strip, however any soft fabric may be used.

At least one of the head garment or back flap may be UV resistant.

The device of the invention may comprise at least one an advertising platform on which advertisements can be placed.

The invention further provides a kit comprising at least one head garment comprising a band of material for surrounding a user's head, and at least two back flaps comprising a head garment-cooperating end attachable to at least one of said head garments and a free end on which the user sits; wherein in use with the band of material surrounding the user's head and the head garment-cooperating end of the back flap attached to the head garment, the back flap is of sufficient length to allow the user to sit on the free end, in order to put the back flap under tension thereby restricting movement of the user's head and neck to stabilise the user's spine.

The kit may comprise two or more back flaps of differing sizes, material, colour or having other distinguishing features.

The back flap has a tapered form.

The head garment may comprise a band of material for surrounding a user's head, and may be adapted to receive a back flap comprising a head garment-cooperating end attachable to the head garment and a free end on which the user sits, to form a cervical spine and neck support device; wherein in use with the band of material surrounding the user's head and the head garment-cooperating end of the back flap attached to the head garment, the back flap is of sufficient length to allow the user to sit on the free end, in order to put the back flap under tension thereby restricting movement of the user's head and neck to stabilise the user's spine.

The back flap may comprise a head garment-cooperating end adapted for attaching to a head garment comprising a band of material for surrounding a user's head, and a free end on which the user sits, wherein in use with the band of material surrounding the user's head and the head garment-cooperating end of the back flap attached to the head garment, the back flap is of sufficient length to allow the user to sit on the free end, in order to put the back flap under tension thereby restricting movement of the user's head and neck to stabilise the user's spine.

The cervical spine and neck support device of the present invention, when in use, holds the spine in its natural position without the need for any further aid. The cervical spine and neck support device further maximises the toad bearing characteristic of the neck by maintaining the neck in its proper structural position. The cervical spine and neck support device of the present invention also prevents slumping occurring in a person wherein the slumping may be caused by excess fatigue.

Hence, the cervical spine and neck support device allows the user to sleep without having to hold themselves up. The cervical spine and neck support device can facilitate a holding pattern which will allow the person to relax by stabilizing the cervical spine. The cervical spine and neck support of the invention further limits the extent of flexion and side bending of the user's spine.

### Brief Description of the Drawings

The invention will now be described with reference to the accompanying drawings in which:
Figure 1 is a side view of a cervical spine and neck support device in accordance with one embodiment of the present invention.
Figure 2 is a rear view of the cervical spine and neck support device of figure 1.
Figure 3 is a rear view of the support device of Figure 1 being worn by a user in a standing position.
Figure 4 is a side view of the user of Figure 3 moving from a standing position to a seated position.
Figure 5 is a side view of the user of figure 3 in a seated position wherein the support supports and stabilises the cervical spine of the user.
Figure 6 is an alternative embodiment of the cervical spine and neck support device of the present invention worn by a user in a standing position.
Figure 7 is a front view of the user of figure 6 in a seated position, demonstrating correct positioning of the support in use.
Figure 8 is a detailed view of the head garment of one embodiment of the support of the present invention.
Figure 9 is a detailed side view of the head garment of an alternative embodiment of the support of the present invention.
Figure 10 is a detailed rear view of the head garment of one embodiment of the support of the present invention.
Figure 11 is a detailed front view of the cervical spine and neck support device of figure 6.

### Detailed Description of the Drawings

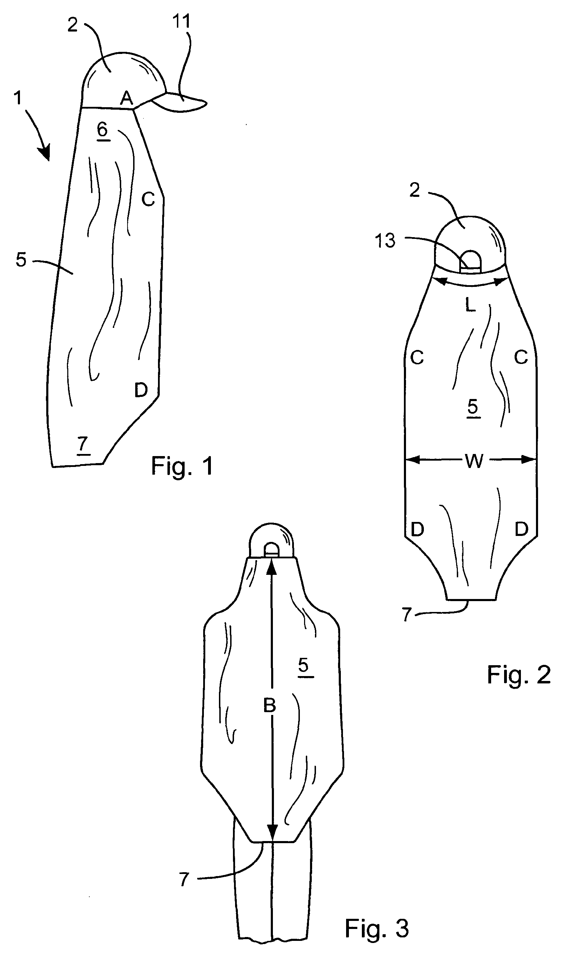

**Figure 1** shows a cervical spine and neck support device 1 in accordance with a first embodiment of the invention. When worn by a user, the support device can be used to stabilise the spinal symmetry of the user when in a seated position.

The cervical spine and neck support device 1 is shown to comprise a head garment 2 and a back flap 5. In the embodiment depicted in figure 1 the head garment is a ball cap, although the head garment 2 is not restricted to being a cap. Any suitable head garment comprising a band of material capable of surrounding /circumventing the user's head can be used, for example any form of hat, band, headband, visor, or any other head gear. The material used for the band can be any material. The cap further comprises a peak or visor 11 that is suitable for acting as a barrier to the sun's rays. It will be appreciated that either the cap or the back flap or both can be UV resistant.

The back flap 5 has an upper end 6 that is attached to the lower edge of the main body of the cap 2, and a lower free end 7.

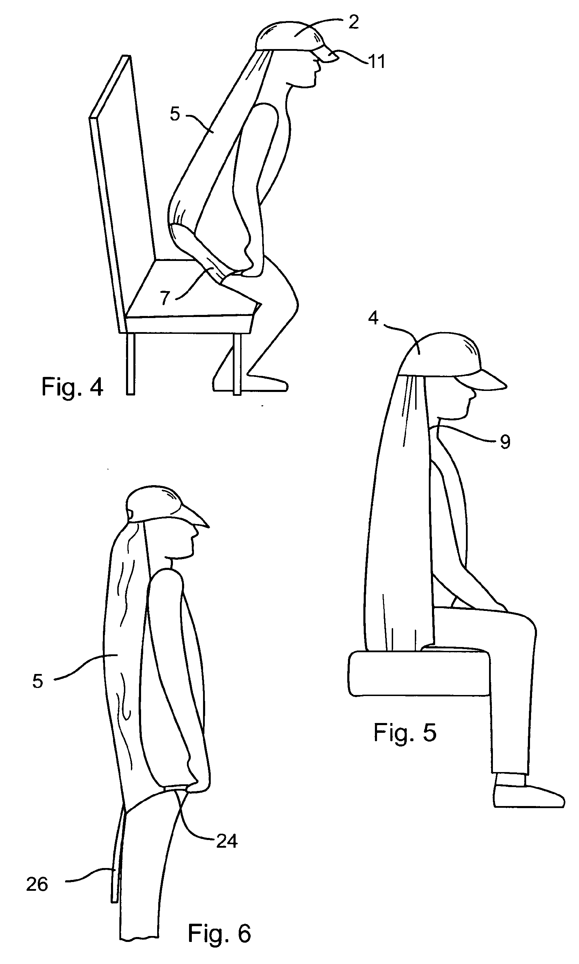

**Figure 3** shows the cervical spine and neck support device of figures 1 and 2 being worn by a standing user. The back flap 5 is of sufficient length B to extend down the wearer's back with the lower free end 7 resting below the user's buttocks. As shown in **figure 4**, this is essential to ensure that the user is able to sit on the lower free end 7 when in a seated position. The length B of the back flap 5 from the base of the cap 2 to its free end 7 is dependent on the intended user's height. For an adult of approximately six foot, the length of the back flap may be approximately 130cm. A shorter back flap would be used for a child.

**Figure 5** shows arrangement of the support once the user has reached an in-use seated position. The support works in combination with the user's body when seated by forming a kind of head, back and spinal brace which holds the head and spine stationary. Figure 4 shows a user preparing to use the support. As the user sits, he/she holds onto each side of the back flap 5 as shown to keep the back flap taught both across its transverse axis and along its longitudinal axis. This ensures that once seated the back flap 5 is put under tension as shown in Figure 5. The taught back flap envelops the user's neck, shoulders and back and provides a downward pull on the cap towards the user's head. This restricts movement of the user's head and neck to stabilise the user's spine. The cervical spine and neck support device 1 limits the extent of flexion and side bending of the user's spine.

If the user begins to slump to the right, the tension on the left hand side of the flap increases. The increase in tension will apply a force that will maintain the user in the upright position. Likewise if the user begins to slump to the left, the tension on the right hand side of the flap increases. Similarly, the portion of the taught back flap 5 running up the user's spine prevents the user's head from rolling forward, while the front edges of the back flap (trapped under the user's thighs) prevent the user's head from rolling backwards.

As shown in Figure 2, the back flap 5 is shaped to best support the spinal column of the user. The overall profile of the back flap 5 combines to allow the stabilizing of the user's whole spinal column.

In the embodiment shown in Figures I and 2, the back flap is attached to the cap's lower band along a line L running between points A on each side of the cap 2. In the embodiment shown (which is sized for an adult) the line L is within the range from about 35-48 cm. Preferably the line L is of length approximately 45 cm. When worn, this positions the start of the back flap just forward of the wearer's ears.

The back flap then gradually tapers outwards to points C to enable the flap to envelope the wearer's shoulders. The width of the flap between the points C is within the range from about 40-60 cm, preferably the width of the flap between points C is approximately 53 cm. The length of the flap that tapers from the cap to point C is within the range of about 10-30cm. Preferably the length of the flap that tapers from the cap to point C is approximately 20cm. By enveloping the wearer's shoulders, particularly in an in use seated position, the taught flap stabilises the wearer's shoulders and prevents the 1^{st} thoracic rib at the base of the neck from going into adaption.

The full width W of the back flap is maintained until a short distance from the free end at points D wherein it tapers again. The preferred distance between point C and D is wi:hin the range of about 60-110cm; most preferably the distance is approximately 85cm. The length of the flap from point D to the end of the free end of the flap 7 is within the range of about 10-40cm; most preferably the length is approximately 35cm. The tapering at the free end 7 of the back flap 5 assists the wearer in pulling the flap taught between his/her legs when getting into a sitting position.

An adjustable strap 13 allows for the tightening or loosening of the cap 2 on the user's head.

As mentioned above, the length of the back flap 5 extends beyond the buttocks of the user. The width of the flap in this region is sufficient to envelop the user's pelvis and buttocks. In use in a seated position, the flap holds both sitting bones (Ischium bones) of the user in tandem with the user's shoulder blades.

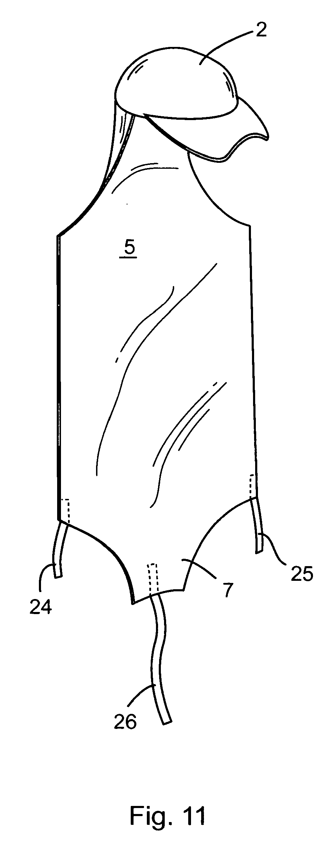

**Figure 6** shows an alternative embodiment of support device. This support has all of the features of the support in figures 1 to 5. In addition, tags are provided on the back flap to assist the user in correctly positioning the back flap prior to sitting down. These tags are more clearly shown in Figure 11. A side tag 24, 25 extends from each side edge of the flap 5 in the region of where the flap begins to taper. These "pelvic side tags" 24 and 25 can be gripped by the wearer in a standing position, as shown in Figure 6, to spread and pull the flap tight across the their buttocks. By doing so prior to sitting, the user can set proper spread of the back flap 5 underneath the buttocks when seated for equal tension and comfort. The back flap 5 provides equal staying tension when spread uniformly under the user. Furthermore the pelvic tags 24 and 25 can be used to eliminate creases in the back flap thereby contributing to comfort once seated.

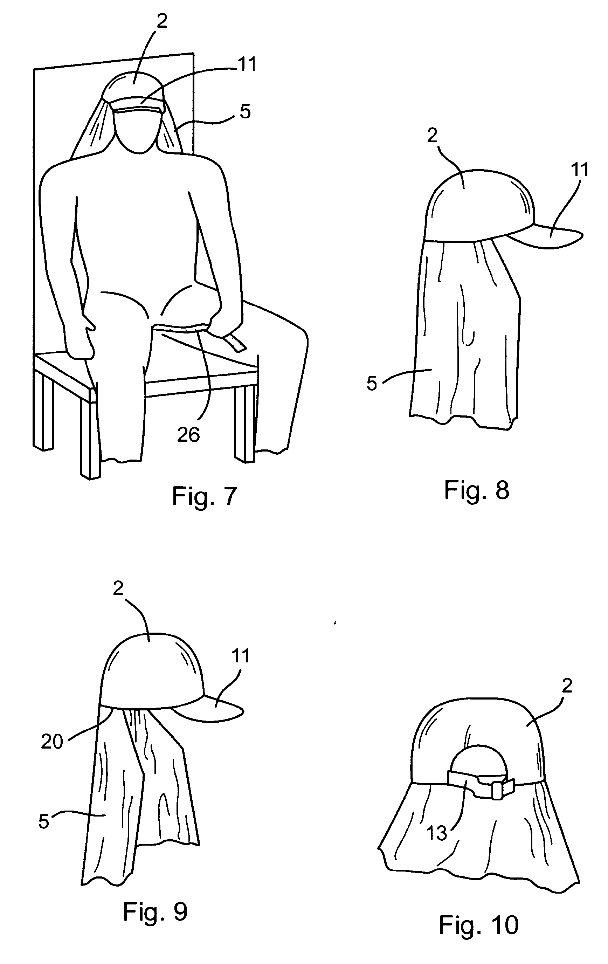

The back flap 5 further comprises a front tag 26, which extends from the centre of the free end of the back flap. As shown in **Figure 7**, once seated, the user can pull on the front tag through his/her legs to pull the back flap under his/her buttocks to create the required tension in the back flap 5.

The front tag 26 may be used to fix the tension of the back flap 5 thereby allowing the user to control and set their desired head tilt position. Pulling the front tag 26 forward will increase the tension on the back flap and on the head garment 2 and will pull the user's head backwards. Relieving the front tag 26 will release tension in the back flap 5 and will allow the user's head to drop towards his/her chest. Ideally, the front tag 26 should be adjusted so that the user's head is maintained in a neutral position supported by the spinal column .

During use, the user's head tilt position may be easily adjusted using the front tag 26 provided. By temporality lifting their body weight from the back flap 5, the user can adjust the tension in the flap using the front tag 26 and the peak 11 until the user's head feels supported in an upright position. In addition to enhancing the comfort and effectiveness of the device, the tags will allow the user to adjust the device with minimum disturbance to other seated persons in the vicinity, for example when on public transport.

**Figure 8** provides a side view of the cap/flap juncture of the cervical spinal supports of Figures 1-7. In use the cap 2 sits on the users head and can be adjusted using the peak 11. In use when worn by a user in a seated position, the user's head is supported utilising the tension of the cap construction over the full surface of the cranial roof of the head, as well as the full tension support of the width of the back flap 5 as it extends fully secured from the base of the cap 2.

In an alternative embodiment as shown in **figure 9**, the back flap 5 can be seen attached to the cap 2 along the base 20 of the cap 2 to just behind the ear.

As seen in **figure 10**, the cap 2 comprises an adjustable strap 13 that allows the internal circumference of the band around the base of the head-receiving main body of the cap 2 to be adjusted according to the user's head size. Additionally, the circumference of the band around the base of the head-receiving main body of the cap 2 may incorporate a comfort strip, which can reduce the circumference of the band to provide a snug fit on the user's head. This comfort strip can also be used to reduce any discomfort which may be caused by friction and the like between the users head and the head garment.

The cap may further comprise one or more pockets for storing accessories such as earplugs or an eye mask, or a personal entertainment device such as a personal music player or games console. It will also be appreciated that the cap may also include audio enhancements such as speakers or headphones to enable the user to use the personal entertainment devices or in-flight or other travel entertainment.

Figure 10 shows the adjustable strap feature 13 of the hat in greater detail. A sufficient amount of flap fabric is maintained between this cap adjustment means to allow for maximum adjustment of the cap without limitation by the flap fabric.

The overall size of the cap will vary depending on the intended wearer. Smaller hats will be provided for children.

Any of the aforementioned embodiments of cap may be also provided with a pocket into which the back flap is packable when not in use. The pocket into which the back flap is packable may be located on the adjustable strap portion. Alternatively the cap may comprise means for supporting a rolled-up back flap, such as a strap which surrounds the rolled up flap. Cooperating means may be provided on both the flap and the head garment which act together to store the flat when not in use. Such means may include buttons or poppers.

In each of the embodiments shown in the drawings, the back flap is integral with the cap. In alternative embodiments, the flap may be releasably attachable to the head garment by releasable attachment means such as a Velcro^{™}, a zip, one or more hook and loops or one or more buttons. In such embodiments, the cap or head garment would resemble any normal cap or head garment when not in use.

The back flap may be interchangeable with an alternative flap. Moreover, different sized, coloured or back flaps of different materials may form part of a pack or kit with one or more head garments.

In the embodiments shown, each edge of the flap is reinforced by an internal cord (not shown), which provides a ridge adjacent the edge of the flap. In use, the ridge provides additional friction against the free end of the back flap slipping out beneath the seated wearer.

The back flap 5 is preferably composed of non-stretch material or minimal stretch material. The flap material should be flexible enough to allow it to conform to the shape of the user's back. Preferably the flap material should feel comfortable against the user's skin so it is conducive to enable to user to relax and sleep. The back flap 5 is usually made from one or more of Silk, Satin, Polyester, Nylon^{™}, Linen, Cotton, Denim, Hemp, Gore-Tex^{™}, PolarTec® Forward Fabric™, thin carbon fiber sheeting woven in fine flexible mesh, Condura^{™} or Kevlar^{™} sheeting.

It will be appreciate that any section of the head garment may be used as an advertising platform.

It will be further appreciated that the use of the support of the invention is not limited to use in aiding sleeping while travelling. The support may be worn whenever correct alignment of the spine is required. It may be used as a training aid for people suffering from bad posture. It is also suitable for use while practicing yoga or meditation without ary back support to promote a healthy posture. It is further suitable for use by children in the back scat of a car with or without the use of child booster seats, It is further suitable for passengers or drivers of any vehicle. The present invention allows the user to relax when in a scated position by stabilising the whole of the spine and pelvis to hold the user's spine in a natural position and preventing slouching into undesirable pain-causing positions.

The words "upper" and "lower" when used herein with reference to the present invention are intended to refer to the orientation of the feature to which they refer when worn by a standing individual. The words "comprises/comprising" and the words "having/including" when used herein with reference to the present invention are used to Specify the presence of stated features, integers, steps or components but does not proclude the presence or addition of one or more other features, integers, steps, components or groups thereof.

## Claims

1. A back flap (5) for use in a cervical spine and neck support device for stabilising the spinal symmetry of a user when seated comprising:
a head garment-cooperating end (6) adapted for attachment to a head garment comprising a band of material for surrounding a user's head, and
a free end (7) on which the user sits,
wherein in use with the band of material surrounding the user's head and the head garment-cooperating end of the back flap attached to the head garment (1), the back flap (5) has a length adapted to allow the user to sit on the free end (7), in order to put the back flap (5) under tension thereby restricting movement of the user's head and neck to stabilise the user's spine; and **characterised in that**
the width of the back flap (5) increases away from its head garment cooperating end to enable the back flap envelope the user's shoulders; and wherein the increased width of the back flap (5) is maintained until a distance from the free end wherein the back flap tapers inwards to assist the user to pull the free end of the flap between his legs when getting into a seated position.

2. A cervical spine and neck support device (1) for stabilising the spinal symmetry of a user when seated comprising:
a head garment (2)comprising a band of material for surrounding a user's head, and
a back flap (5) according to Claim 1.

3. A cervical spine and neck support device (1) as claimed in claim 2 wherein the back flap (5) is removable from the head garment (2).

4. A cervical spine and neck support device (1) as claimed in claim 2 wherein the back flap (5) is permanently attached to the head garment (2).

5. A cervical spine and neck support device (1) as claimed in any of Claims 2 to 4 wherein the head garment (2) is a cap.

6. A cervical spine and neck support device (1) as claimed in any of Claims 2 to 5 further comprising means for storing said back flap (5) when not in use.

7. The cervical spine and neck support device (1) as claimed in any of Claims 2 to 6 further comprising at least one tag (24, 25, 26) on said back flap for aiding correct positioning of the back flap by the user prior to sitting down.

8. The cervical spine and neck support device (1) of any of Claims 2 to 7 wherein the head garment (2) is adjustable to fit the user's head.

9. The cervical spine and neck support device (1) of any of Claims 2 to 8 wherein at least one comfort strip is incorporated in the head garment (2), wherein the comfort strip is a strip of soft fabric extending around any part or all of the band of material for surrounding the user's head.

10. A kit comprising
at least one head garment (2) comprising a band of material for surrounding a user's head, and
at least two back flaps (5) according to Claim 1.

11. The kit as claimed in claim 10 wherein the kit comprises two or more back flaps (5) of differing sizes.

## Patentansprüche

1. Ein Rückenteil (5) zur Verwendung bei einer Vorrichtung zum Stützen von Halswirbelsäule und Nacken zur Stabilisierung der Wirbelsäulensymmetrie eines Trägers im Sitzen, das umfasst:
ein mit einer Kopfbekleidung zusammenwirkendes Ende (6), das so gestaltet ist, dass es an einer Kopfbekleidung befestigt werden kann, welche ein Materialband zum Umschließen des Kopfes eines Trägers umfasst, und
ein freies Ende (7), auf dem der Träger sitzt,
wobei das Rückenteil (5), wenn es mit dem Materialband, das den Kopf des Trägers umschließt, und dem mit der Kopfbekleidung zusammenwirkenden Ende des Rückenteils, das an der Kopfbekleidung (1) befestigt ist, verwendet wird, eine Länge besitzt, die so gestaltet ist, dass es dem Träger möglich ist, auf dem freien Ende (7) zu sitzen, damit das Rückenteil (5) unter Spannung gesetzt wird, so dass die Bewegung des Kopfes und Nackens des Trägers eingeschränkt wird und die Wirbelsäule des Trägers stabilisiert wird, und das **dadurch gekennzeichnet ist, dass**
die Breite des Rückenteils (5) mit steigender Entfernung von dessen Ende, das mit der Kopfbekleidung zusammenwirkt, zunimmt, so dass das Rückenteil die Schultern des Trägers umhüllen kann, und wobei die erhöhte Breite des Rückenteils (5) erhalten bleibt bis zu einer Entfernung zum freien Ende, bei der das Rückenteil sich nach innen verjüngt, damit es für den Träger leichter wird, das freie Ende des Teils zwischen seine Beine zu ziehen, wenn er eine Sitzposition einnimmt.

2. Eine Vorrichtung (1) zum Stützen von Halswirbelsäule und Nacken zur Stabilisierung der Wirbelsäulensymmetrie eines Trägers im Sitzen, umfassend:
eine Kopfbekleidung (2), die ein Materialband zum Umschließen des Kopfes eines Trägers umfasst, und
ein Rückenteil (5) gemäß Anspruch 1.

3. Eine Vorrichtung (1) zum Stützen von Halswirbelsäule und Nacken wie in Anspruch 2 beansprucht, wobei sich das Rückenteil (5) von der Kopfbekleidung abnehmen lässt.

4. Eine Vorrichtung (1) zum Stützen von Halswirbelsäule und Nacken wie in Anspruch 2 beansprucht, wobei das Rückenteil (5) dauerhaft an der Kopfbekleidung (2) befestigt ist.

5. Eine Vorrichtung (1) zum Stützen von Halswirbelsäule und Nacken wie in einem der Ansprüche 2 bis 4 beansprucht, wobei die Kopfbekleidung (2) eine Kappe ist.

6. Eine Vorrichtung (1) zum Stützen von Halswirbelsäule und Nacken wie in einem der Ansprüche 2 bis 5 beansprucht, die ferner Mittel zur Aufbewahrung des Rückenteils (5) umfasst, wenn dieses nicht verwendet wird.

7. Die Vorrichtung (1) zum Stützen von Halswirbelsäule und Nacken wie in einem der Ansprüche 2 bis 6 beansprucht, die ferner wenigstens einen Hänger (24, 25, 26) an dem Rückenteil umfasst, damit der Träger vor dem Hinsetzen das Rückenteil leichter in die richtige Position bringen kann.

8. Die Vorrichtung (1) zum Stützen von Halswirbelsäule und Nacken nach einem der Ansprüche 2 bis 7, wobei die Kopfbekleidung (2) verstellbar ist, um sie an den Kopf des Trägers anzupassen.

9. Die Vorrichtung (1) zum Stützen von Halswirbelsäule und Nacken nach einem der Ansprüche 2 bis 8, wobei wenigstens ein Komfortstreifen in der Kopfbekleidung (2) integriert ist, wobei der Komfortstreifen ein Streifen aus weichem Gewebe ist, der sich um einen beliebigen Teil oder das gesamte Materialband zum Umschließen des Kopfes des Trägers erstreckt.

10. Ein Kit, umfassend
wenigstens eine Kopfbekleidung (2), die ein Materialband zum Umschließen des Kopfes eines Trägers umfasst, und
wenigstens zwei Rückenteile (5) gemäß Anspruch 1.

11. Der wie in Anspruch 10 beanspruchte Kit, wobei der Kit zwei oder mehrere Rückenteile (5) unterschiedlicher Größe umfasst.

## Revendications

1. Pan dorsal (5) destiné à être utilisé dans un dispositif de support de colonne cervicale et de cou pour stabiliser la symétrie spinale d'un utilisateur lorsqu'il est assis, comprenant :
une extrémité coopérant avec un vêtement de tête (6) conçue pour être fixée à un vêtement de tête comprenant une bande de matière destinée à entourer la tête d'un utilisateur, et
une extrémité libre (7) sur laquelle s'assoit l'utilisateur,
dans lequel, lorsqu'il est utilisé avec la bande de matière entourant la tête de l'utilisateur et avec l'extrémité coopérant avec le vêtement de tête du pan dorsal fixée au vêtement de tête (1), le pan dorsal (5) a une longueur adaptée pour permettre à l'utilisateur de s'asseoir sur l'extrémité libre (7), afin de mettre le pan dorsal (5) sous tension, limitant ainsi le mouvement de la tête et du cou de l'utilisateur pour stabiliser la colonne vertébrale de l'utilisateur ; et **caractérisé en ce que**
la largeur du pan dorsal (5) augmente en s'éloignant de son extrémité coopérant avec le vêtement de tête pour permettre au pan dorsal d'envelopper les épaules de l'utilisateur ; et dans lequel la largeur accrue du pan dorsal (5) est maintenue jusqu'à une distance de l'extrémité libre à laquelle le pan dorsal se réduit progressivement vers l'intérieur pour aider l'utilisateur à tirer l'extrémité libre du pan entre ses jambes lorsqu'il se met en position assise.

2. Dispositif de support de colonne cervicale et de cou (1) pour stabiliser la symétrie spinale d'un utilisateur lorsqu'il est assis comprenant :
un vêtement de tête (2) comprenant une bande de matière destiné à entourer la tête d'un utilisateur, et
un pan dorsal (5) selon la revendication 1.

3. Dispositif de support de colonne cervicale et de cou (1) selon la revendication 2, dans lequel le pan dorsal (5) peut être retiré du vêtement de tête (2).

4. Dispositif de support de colonne cervicale et de cou (1) selon la revendication 2, dans lequel le pan dorsal (5) est fixé de façon permanente au vêtement de tête (2).

5. Dispositif de support de colonne cervicale et de cou (1) selon l'une quelconque des revendications 2 à 4, dans lequel le vêtement de tête (2) est une casquette.

6. Dispositif de support de colonne cervicale et de cou (1) selon l'une quelconque des revendications 2 à 5, comprenant en outre des moyens de stockage dudit pan dorsal (5) lorsqu'il n'est pas utilisé.

7. Dispositif de support de colonne cervicale et de cou (1) selon l'une quelconque des revendications 2 à 6, comprenant en outre au moins une étiquette (24, 25, 26) sur ledit pan dorsal pour faciliter le positionnement correct du pan dorsal par l'utilisateur avant qu'il ne s'assoie.

8. Dispositif de support de colonne cervicale et de cou (1) selon l'une quelconque des revendications 2 à 7, dans lequel le vêtement de tête (2) est ajustable pour s'adapter à la tête de l'utilisateur.

9. Dispositif de support de colonne cervicale et de cou (1) selon l'une quelconque des revendications 2 à 8, dans lequel au moins une bande de confort est incorporée dans le vêtement de tête (2), dans lequel la bande de confort est une bande de tissu souple s'étendant autour d'une partie ou de l'ensemble de la bande du matière destinée à entourer la tête de l'utilisateur.

10. Kit comprenant
au moins un vêtement de tête (2) comprenant une bande de matière destinée à entourer la tête d'un utilisateur, et
au moins deux pans dorsaux (5) selon la revendication 1.

11. Kit selon la revendication 10, dans lequel le kit comprend deux pans dorsaux ou plus (5) de tailles différentes. *ₑₚ₁₆₇₂₆*
